# EUROPEAN PATENT SPECIFICATION

(11) **EP 2 229 128 B1**
(45) Date of publication and mention of the grant of the patent: **30.09.2015**
(21) Application number: 08858223.4
(22) Date of filing: 27.10.2008
(51) Int. Cl.: A61F 5/00

(54) **POLYMERIC MICROSPHERES FOR TREATMENT OF OBESITY**
POLYMER-MIKROSPHÄREN ZUR BEHANDLUNG VON ADIPOSITAS
MICROSPHÈRES POLYMÈRES POUR LE TRAITEMENT DE L'OBÉSITÉ

(30) Priority: 05.12.2007 US 992483 P; 21.10.2008 US 255271
(43) Date of publication of application: 22.09.2010
(73) Proprietor: Boston Scientific Scimed, Inc., Maple Grove, MN 55311-1566 (US)
(72) Inventor: KAUL, Goldi, Nyack, New York 10960 (US); KRUEGER, Katie L., Merrimack, New Hampshire 03054 (US)
(74) Representative: Vossius & Partner Patentanwälte Rechtsanwälte mbB
(86) International application number: PCT/US2008/081336
(87) International publication number: WO 2009/073293

(56) References cited:
- US-A1- 2004 037 865
- US-A1- 2004 089 313
- US-A1- 2004 253 274
- US-A1- 2006 088 568
- US-A1- 2007 078 435

## Description

### BACKGROUND

Surgical treatments for morbid obesity are often necessary when approaches such as lifestyle changes such as reduced calorie intake with or without appetite suppressing medication and/or increased exercise are unsuccessful. These procedures may include gastric bypass such as the Roux-En-Y procedure, gastric banding, implantation of stimulation devices, or placing mechanical restrictions in the stomach. Many of the approaches reduce the effective size of the stomach, fill part of the stomach with a restriction, or artificially cause the patient to feel full. Alternatively, the flow of food into the intestines is reduced, so that fewer calories are absorbed. Surgical methods generally reduce caloric intake by either impeding absorption of ingested calories or reducing the amount of food required to cause feelings of satiety.

Current surgical treatments for obesity often involve invasive, open surgery which is painful and which may entail serious side effects and significant recovery times. Even at expert centers dedicated to carrying out these procedures, the mortality rate may be approximately 0.5%. Additional drawbacks include staple and/or suture line leakage, ulcers forming at gastrojejunal anastomoses, long term nutritional deficiencies, port problems, band slipping, and band erosion.
From document US 2004/0037865 A1 a method of controlling obesity by physically narrowing the lumen of the pylorus has become known. The known method includes injection of a bio-compatible bulking or stiffening material into the pyloric sphincter area of the stomach.
From document US 2007/0078435 A1 an injectable compound has become known including a microsphere-based filler material. The document also mentions filler materials with adhesive properties, for example microparticles made of biological materials such as collagen or hyaluronic acid.

### SUMMARY OF THE INVENTION

Described herein according to one embodiment is a system for treating obesity according to claim 8.

Another embodiment is directed to an injectable compound for treating obesity, according to claim 1.

### BRIEF DESCRIPTION OF THE DRAWINGS

Figure 1 is a diagram showing a side view of a stomach with the pyloric sphineter where the procedure according to the present invention is carried out; and
Figure 2 is a cross sectional view on line II-II showing the pyloric sphincter of Fig. 1 and an injection device according to the present invention.

### DETAILED DESCRIPTION

The present invention may be further understood with reference to the following description and to the appended drawings, wherein like elements are referred to with the same reference numerals. The present invention relates to devices for treating morbid obesity. In particular, the present invention relates to changing the properties of the pyloric sphincter by introducing a therapeutic agent thereinto to delay gastric emptying, expedite satiety and decrease food consumption.

The embodiments of the present invention provide systems to treat obesity by delaying the emptying of contents of the stomach into the intestines, thus leading to early and prolonged satiety and reduced food intake. The exemplary procedures are less invasive than prior obesity treatments as they may be limited to the injection of a therapeutic compound containing microspheres into the pyloric muscle. More specifically, in the present invention, the microspheres are administered intramuscularly into the pyloric muscle in the vicinity of the pyloric sphincter to affect the behavior of the sphincter.

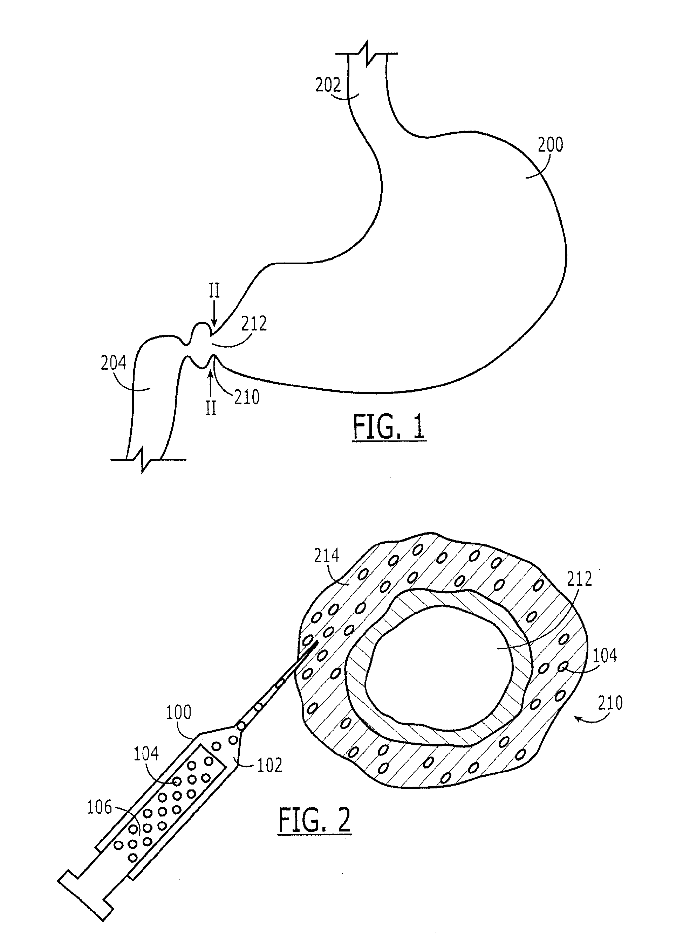

As shown in Figs. 1 and 2, the distal end of the stomach 200 is connected to the duodenum 204 by the pylorus 214 which is separated from the stomach by the pyloric sphincter 210. Partially digested food, or chyme, passes through the pyloric sphincter 210 to continue digestion in the intestines. By controlling the operation of the pyloric sphincter 210 it is possible to control the rate at which food passes into the intestines. Retaining food in the stomach 200 longer reduces the rate at which space in the stomach 200 can be freed for more food and brings on satiety sooner.

Multiple polymeric microspheres 104 are injected into the pyloric sphincter 210 to alter the compliance of the sphincter 210. The microspheres 104 are combined with a carrying fluid (e.g., hyaluronic acid, saline solution, etc.) to form an injectable compound 106. The polymeric microspheres 104 are preferably selected to have dimensions and properties of compressibility and rigidity so that the injectable compound 106 generates desired changes on the tissue into which it is injected. For example, the microspheres 104 will preferably have a compressibility and a rigidity sufficient to withstand peristaltic movements of the GI tract and to reduce the compliance of the pyloric sphincter 210 to a desired degree. The total injection volume may vary between approximately 0.25ml and 30ml. The number of microspheres used for the procedure would depend on the condition and the nature of narrowing that the physician wants to achieve. The density of the microspheres would depend on the manner in which the microspheres are prepared. Porous microspheres may be prepared having lower densities than their non-porous counterparts. Densities of the microspheres would typically range from 0.2 - 1.5 g/cc. Skeletal density of the microspheres would also depend on the polymer type used for their synthesis. For this particular application, two more important properties are compressibility and rigidity of the microspheres. A preferred implementation incorporates a rigid microsphere that compresses sufficiently enough to be delivered through a needle but at the same time retains its inherent shape and size after delivery into the muscle.

Examples of suitable non-degradable polymers include polyhydroxyl methacrylates (polyHEMAs), carbohydrates, polyacrylic acids, polymethacrylic acids, polyvinyl sulfonates, carboxymethyl celluloses, hydroxyethyl celluloses, substituted celluloses, polyacrylamides, polyamides, polyureas, polyurethanes, polyesters, polyethers, polysaccharides, polylactic acids, polymethylmethacrylates, polycaprolactones, polyglycolic acids, polylactic-co-glycolic acids (e.g., polyd-lactic-co-glycolic acids) and copolymers or mixtures thereof. Examples of biodegradable polymers include PLAs, PGAs, polycaprolactones (e.g., poly-M-caprolactone), polyglycolic acids, polylactic-co-glycolic acids (e.g., polyd-lactic-co-glycolic acids, poly lactic acid (e.g., poly-L-lactic acid, poly-D,L-lactic acid), poly-p-dioxanones, polytri-methylene carbonates, polyanhydrides, polyortho esters, polyurethanes, polyamino acids, polyhydroxy alcanoates, polyphosphazenes, poly-b-malein acids, collagen (proteins), chitin, chitosan (polysaccharides), fibrin and albumin. Examples of techniques used to make suitable microspheres include methods shown in Tables I and II below.

**Table-I: Chemical Processes for Microsphere fabrication**

| **Chemical processes** | | |
|---|---|---|
| S. No | Process Type (Polymer) | Suspending medium |
| 1. | Complex coacervation (*Water soluble polyelectrolyte)* | Water |
| 2. | Coacervation by polymer-polymer incompatibility *(Hydrophilic or hydrophobic polymers)* | Organic solvent |
| 3. | Interfacial polymerization at liquid-liquid and solid-liquid interfaces (*Water soluble and insoluble monomers)* | Aqueous/organic solvent |
| 4. | *In situ* polymerization (*Water soluble and insoluble monomers)* | Aqueous/organic solvent |
| 5. | Solvent evaporation or in-liquid drying *(Hydrophilic or hydrophobic polymers)* | Aqueous/organic solvent |
| 6. | Thermal or ionic gelation (*Hydrophilic or hydrophobic polymers)* | Organic |
| 7. | Desolvation in liquid media (*Hydrophilic or hydrophobic polymers)* | Aqueous/organic solvent |
| 8. | Super critical fluid technology | Aqueous/organic solvent |

**Table-II: Mechanical Processes for Microsphere fabrication**

| **Mechanical Processes** | |
|---|---|
| **S.No.** | Process Type (Polymer) |
| 1. | Spray drying |
| | *(Hydrophilic or hydrophobic polymers)* |
| 2. | Spray chilling |
| | *(Hydrophilic or hydrophobic polymers)* |
| 3. | Fluidized bed drying |
| | *(Hydrophilic or hydrophobic polymers)* |
| 4. | Electrostatic deposition |
| 5. | Centrifugal extrusion |
| 6. | Interfacial polymerization at solid-gas or liquid-gas interfaces |
| 7. | Spinning disk |
| 8. | Extrusion or spraying into a desolvation bath |

As described above, by altering the compliance and size of opening of the pyloric sphincter 210, gastric emptying of the chyme into the duodenum 204 is restricted retaining increased volumes of food in the stomach 200 and expediting and prolonging satiety. That is, the injected polymeric microspheres 104 not only increase the resistance of the sphincter 210 to opening, they also bulk the tissue of the pyloric muscle 214 reducing an area of a lumen 212 through the sphincter 210.

The polymeric microspheres 104 may be administered to the patient through an intramuscular injection into the circular and/or longitudinal pyloric muscle 214, for example using an injection device 102 which may be inserted into the stomach 200 via the esophagus using an endoscope (not shown) as would be understood by those skilled in the art. The injection device 102 may, for example, be similar to a sclerotherapy needle which would be compatible with a conventional syringe. The distal tip of the device would incorporate a needle for penetrating the pyloric wall from the inner lumen of the GI tract. Alternatively the microspheres may be injected laproscopically. An injection device could be delivered through the laparoscopic port and the penetrating needle of the device would enter the external surface of the pyloric wall to deliver the microspheres.

Those of skill in the art will understand that the spatial placement of the microspheres 104 into the pylorus 214 may be varied to suit different applications and to obtain desired therapeutic effects. For example, the microspheres 104 may be administered as multiple boluses injected at a plurality of locations spaced circumferentially around the pyloric muscle 214. Alternatively, the microspheres 104 may be injected as a single bolus in one location to localize the reduced compliance at a desired location radially around the sphincter 210 and/or to generate a portion of the sphincter 210 which projects into the lumen 212 at the radial location.

In another embodiment according to the invention, the microspheres 104 may provide additional functionalities. For example, radiopaque elements or a fluoroprobe may be included in the injectable compound 106 or as part of the microspheres 104 to facilitate visualization of the deployed microspheres 106 within the pylorus 214 using a fluoroscope, an endoscope and/or a CT scanner. Visual markers may also be included in all or a portion of the microspheres 104, to permit visual observation of the deployment pattern.

One or more therapeutic agents may be added to the microspheres 104 or to the injectable compound 106 for treatment of the tissue into which the microspheres 104 are injected. For example, the microspheres104 may be coated with a therapeutic agent which facilitates the generation of satiety signals or which reduces the number of calories extracted from ingested food as would be understood by those skilled in the art. Alternatively or additionally, the therapeutic agent may be added to the carrying fluid which, along with the microspheres 104, forms the injectable compound 106. As would be understood by those skilled in the art, other therapeutic agents may be added to the injectable compound 106 as necessary to achieve therapeutic goals. Furthermore, the exemplary microspheres 104 may be formed to exhibit bio-adhesive properties to enhance their attachment to the tissue of the pyloric muscle 214 making migration of the microspheres 104 away from the injection location less likely. For example, a bio-adhesive coating may be provided including any of polymers such as poly acrylic acid, polyethylene glycol, polyN-vinyl, 2-pyrollidone, hyaluronic acid, hydroxyethyl cellulose, methylcellulose, pectin, carboxy methyl cellulose, alginates, chitosan, gelatin, dextrans etc. Alternatively, the microspheres may be coated with a material promoting scarring to tighten the sphincter to promote fibrin encapsulation or mucal deposits to further tighten the sphincter.

The system according to the present invention allows the microspheres 104 to be delivered directly into muscle (such as the pyloric muscle 214) to enhance packing while reducing slip planes which allows users greater control of the type and degree of the alteration in the properties of the pyloric sphincter 210. The substantially spherical shape of the microspheres 104 reduce muscle trauma achieving a corresponding reduction in discomfort and side effects as would be understood by those skilled in the art. In addition, as the sphincter 210 is accessed via an endoscope inserted via a naturally occurring body orifice (i.e., the mouth) the only penetration of tissue required is the piercing of the sphincter 210 by the injection device 102. Thus, the discomfort, complications and extended recovery times associated with open surgery are avoided.

The present invention has been described with reference to specific exemplary embodiments. Those skilled in the art will understand that changes may be made in details, particularly in matters of shape, size, material and arrangement of parts. Accordingly, various modifications and changes may be made to the embodiments. The specifications and drawings are, therefore, to be regarded in an illustrative rather than a restrictive sense.

## Claims

1. An injectable compound (106) for treating obesity, comprising:
a fluid carrier injectable intramuscularly into one of a pyloric sphincter (210) and tissue adjacent to a pyloric sphincter; and
a plurality of microspheres (104) suspended in the fluid carrier and adapted to alter one of a compliance
of the pyloric sphincter (210) and a lumen area of the pyloric sphincter (210), wherein the microspheres (104) comprise a bio adhesive, **characterized in that** the microspheres (104) include at least one of a radiopaque element, a fluoroprobe, and a visual marker.

2. The injectable compound (106) according to claim 1, wherein the bio adhesive is provided as a coating including a polymer selected from the group consisting of poly acrylic acid, polyethylene glycol, polyN-vinyl, 2-pyrollidone, hyaluronic acid, hydroxyethyl cellulose, methylcellulose, pectin, carboxy methyl cellulose, alginates, chitosan, gelatin, and dextrans.

3. The injectable compound (106) according to claim 1, wherein the microspheres (104) include an agent for treatment of obesity.

4. The injectable compound (106) according to claim 1, wherein the microspheres (104) are polymeric microspheres.

5. The injectable compound (106) according to claim I, wherein the fluid carrier comprises one of saline and hyaluronic acid.

6. The injectable compound (106) according to claim 1, wherein the microspheres (104) have a density of about 0.2 g/cc to 1.5 g/cc.

7. The injectable compound 106 according to claim 1, wherein the microspheres (104) have diameters between about 100 microns and 5,000 microns.

8. A system for treating obesity including the injectable compound (106) of claim 1, further comprising:
an injection device (102) including a distal portion adapted for insertion to a target injection site one of adjacent to and in the pyloric sphincter (210).

9. The system according to claim 8, wherein the injection device 102 has a flexibility sufficient to pass through a natural body lumen into the stomach (200) to the target injection site.

10. The system according to claim 8, wherein the injection device (102) is sized to be slidably received in a working channel of one of an endoscope and a laparoscope.

11. The system according to claim 8, wherein the injection device (102) includes a tissue piercing tip and a lumen through which the injectable (106) compound may be delivered.

12. The system according to claim 8, wherein the microspheres (104) have compressibility and rigidity sufficient to withstand peristaltic movement of the GI tract.

13. The system according to claim 8, wherein the injection device (102) comprises a syringe.

14. The system according to claim 8, wherein the system is adapted to administer the injectable compound (106) as a single bolus or the system is adapted to administer the injectable compound (106) as multiple boluses.

## Patentansprüche

1. Eine injizierbare Verbindung (106) zur Behandlung von Adipositas, umfassend:
einen flüssigen Träger, der in einen/eines von einem *Sphincter pylori* (210) und Gewebe angrenzend an einen *Sphincter pylori* intramuskulär injizierbar ist; und
eine Vielzahl von Mikrosphären (104), die in dem flüssigen Träger suspendiert sind und angepasst sind, um eine/eines von der Nachgiebigkeit des *Sphincter pylori* (210) und einer Lumenfläche des *Sphincter pylori* (210) zu verändern, wobei die Mikrosphären (104) einen Bioklebstoff umfassen, der **dadurch gekennzeichnet ist, dass** die Mikrosphären (104) mindestens eines/eine/einen aus einem radiopaken Element, einer Fluoreszenzsonde und einem visuellen Marker enthalten.

2. Die injizierbare Verbindung (106) gemäß Anspruch 1, wobei der Bioklebstoff als ein Überzug bereitgestellt ist, der ein Polymer enthält, ausgewählt aus der Gruppe bestehend aus Polyacrylsäure, Polyethylenglycol, PolyN-vinyl, 2-Pyrrolidon, Hyaluronsäure, Hydroxyethylcellulose, Methylcellulose, Pectin, Carboxymethylcellulose, Alginaten, Chitosan, Gelatine und Dextranen.

3. Die injizierbare Verbindung (106) gemäß Anspruch 1, wobei die Mikrosphären (104) ein Mittel für die Behandlung von Adipositas enthalten.

4. Die injizierbare Verbindung (106) gemäß Anspruch 1, wobei die Mikrosphären (104) Polymer-Mikrosphären sind.

5. Die injizierbare Verbindung (106) gemäß Anspruch 1, wobei der flüssige Träger eines von Saline und Hyaluronsäure umfasst.

6. Die injizierbare Verbindung (106) gemäß Anspruch 1, wobei die Mikrosphären (104) eine Dichte von etwa 0,2 g/cc bis 1,5 g/cc haben.

7. Die injizierbare Verbindung (106) gemäß Anspruch 1, wobei die Mikrosphären (104) Durchmesser zwischen etwa 100 Mikrometer und 5000 Mikrometer haben.

8. Ein System zur Behandlung von Adipositas, das die injizierbare Verbindung (106) aus Anspruch 1 enthält, weiter umfassend:
eine Injektionsvorrichtung (102), die einen distalen Abschnitt enthält, der angepasst ist für die Insertion in einer Zielinjektionsstelle, die eines aus benachbart zu und in dem *Sphincter pylori* (210) ist.

9. Das System gemäß Anspruch 8, wobei die Injektionsvorrichtung 102 eine Flexibilität aufweist, die ausreicht, um durch ein natürliches Körperlumen in den Magen (200) zu der Zielinjektionsstelle zu führen.

10. Das System gemäß Anspruch 8, wobei die Injektionsvorrichtung (102) so bemessen ist, dass sie verschiebbar in einem Arbeitskanal von einem aus einem Endoskop und einem Laparoskop aufgenommen werden kann.

11. Das System gemäß Anspruch 8, wobei die Injektionsvorrichtung (102) eine Gewebedurchstechspitze und ein Lumen, durch das die injizierbare (106) Verbindung abgegeben werden kann, enthält.

12. Das System gemäß Anspruch 8, wobei die Mikrosphären (104) eine Kompressibilität und Festigkeit aufweisen, die ausreichen, um peristaltischen Bewegungen des Gastrointestinaltrakts standzuhalten.

13. Das System gemäß Anspruch 8, wobei die Injektionsvorrichtung (102) eine Spritze umfasst.

14. Das System gemäß Anspruch 8, wobei das System angepasst ist, um die injizierbare Verbindung (106) in einem einzigen Bolus zu verabreichen oder das System angepasst ist, um die injizierbare Verbindung (106) als mehrere Boli zu verabreichen.

## Revendications

1. Composé injectable (106) pour traiter l'obésité, comprenant :
un véhicule liquide injectable par voie intramusculaire dans le sphincter du pylore (210) et/ou le tissu adjacent au sphincter du pylore ; et
une pluralité de microsphères (104) en suspension dans le véhicule liquide et adaptées pour modifier la compliance du sphincter du pylore (210) et/ou une zone de lumière du sphincter du pylore (210), dans lequel les microsphères (104) comprennent un bio-adhésif, **caractérisé en ce que** les microsphères (104) contiennent au moins un élément radio-opaque, et/ou une sonde fluorée, et/ou un marqueur visuel.

2. Composé injectable (106) selon la revendication 1, dans lequel le bioadhésif est fourni sous la forme d'un revêtement comprenant, un polymère choisi dans le groupe constitué par l'acide polyacrylique, le polyéthylène glycol, le polyN-vinyle, la 2-pyrollidone, l'acide hyaluronique, l'hydroxyéthylcellulose, la méthylcellulose, la pectine, la carboxyméthylcellulose, les alginates, le chitosane, la gélatine et les dextranes.

3. Composé injectable (106) selon la revendication 1, dans lequel les microsphères (104) contiennent un agent pour le traitement de l'obésité.

4. Composé injectable (106) selon la revendication 1, dans lequel les microsphères (104) sont des microsphères polymères.

5. Composé injectable (106) selon la revendication 1, dans lequel le véhicule liquide comprend une solution salée et/ou un acide hyaluronique.

6. Composé injectable (106) selon la revendication 1, dans lequel les microsphères (104) ont une densité d'environ 0,2 g/cc à 1,5 g/cc.

7. Composé injectable (106) selon la revendication 1, dans lequel les microsphères (104) ont des diamètres entre environ 100 microns et 5000 microns.

8. Système pour traiter l'obésité contenant le composé injectable (106) selon la revendication 1, comprenant en outre :
un dispositif d'injection (102) comportant une partie distale adaptée pour l'insertion dans un site d'injection cible, adjacent au sphincter du pylore et/ou dans le sphincter du pylore (210).

9. Système selon la revendication 8, dans lequel le dispositif d'injection 102 a une flexibilité suffisante pour atteindre le site d'injection cible en pénétrant dans l'estomac (200) par une lumière corporelle naturelle.

10. Système selon la revendication 8, dans lequel le dispositif d'injection (102) est dimensionné pour être reçu par coulissement dans un canal de travail d'un endoscope et/ou d'un laparoscope.

11. Système selon la revendication 8, dans lequel le dispositif d'injection (102) comporte une pointe de percement de tissu et une lumière par laquelle le composé injectable (106) peut être administré.

12. Système selon la revendication 8, dans lequel les microsphères (104) ont une compressibilité et une rigidité suffisantes pour résister au mouvement péristaltique du tractus gastro-intestinal GI.

13. Système selon la revendication 8, dans lequel le dispositif d'injection (102) comprend une seringue.

14. Système selon la revendication 8, dans lequel le système est adapté pour administrer le composé injectable (106) sous la forme d'un bolus unique ou le système est adapté pour administrer le composé injectable (106) sous la forme de bolus multiples.
